# EUROPEAN PATENT APPLICATION

(11) **EP 3 269 798 A1**
(43) Date of publication of application: **17.01.2018**
(21) Application number: 16773106.6
(22) Date of filing: 31.03.2016
(51) Int. Cl.: C12M 3/00, C12M 1/00

(54) **INCUBATOR PROVIDED WITH CLEAN BENCH FUNCTION, AND INCUBATOR SYSTEM**

(30) Priority: 31.03.2015 JP 2015070973
(71) Applicant: Dokkyo Medical University, Shimotsuga-gun, Tochigi 321-0293 (JP)
(72) Inventor: CHIKUDA Makoto, Saitama 343-8555 (JP); YOSHIDA Shinichiro, Koshigaya-shi Saitama 343-8555 (JP); OKAMOTO Hiroyuki, Koshigaya-shi Saitama 343-8555 (JP); ARAI Kiyomi, Koshigaya-shi Saitama 343-8555 (JP)
(74) Representative: Naylor, Matthew John
(86) International application number: PCT/JP2016/060644
(87) International publication number: WO 2016/159226

(57) **Abstract**

The present invention provides an incubator with a clean bench function to have both function of opened and closed system and a proposal how to use, allowing cells or others to be "closable and openable" manipulated and cultured, as well as an incubator, a globe box, a clean bench. This is implemented in this system has a specific structure and a characteristic component, e.g., a structure classified two units by the inside temperature, an operation cover and a circular fixture, a channel from/to inside or outside etc. to allow such as used, a non-opening operation and supply and observation from outside, or a moving of perfusion culture device and medium storing bags kept on connecting tubes or others. An incubator 100 with a clean bench function mainly includes a chamber 110, 210, 220, 230, a temperature control unit 160, 213 and a gas concentration control and supply unit 140, 240, as well as a CO₂ cylinder 170, a N₂ cylinder 180, and a N₂ gas generator 190. The chamber 210 has an ability of setting on low temperature. The chamber 110 has the shape of a rectangular parallelepiped and includes an opening 111 in the front, and a sensor 161. The opening 111 attached on a sealing door 117 and an inner door 118, and their intervening space with elastic materials 120-122 and channels 126-128 in a peripheral portion 112 around the opening 111. For the closable operation inside the chamber 110 from the outside, an operation cover 136 and a circular fixture 300, a membrane with/without slit 134 and the fixer 134a, b is attached on/off the fixing hole 119 in the inner door 118, and a first hook 114 to hang of the operation cover is attached to each of two inner side surfaces 113 of the chamber 110. A UV lamp 116 adapted to emit UV rays for sterilization is attached on upper site of inner back surface 115 in the chamber 110. The N₂ gas supplied in large quantity into the chamber from N₂ cylinder 180 that positive pressure is maintained in the chamber 110 relative to outside pressure as used the clean bench function. In particular, this present invention is possible to mostly three new matters as follows: First, the exchange and regulate with gas composition and temperature in this system is both of them different constituent at the same time. Second, this system is an ability to operate under the condition of very low temperature (from 4 degree Celsius) and low O₂ concentration (from 1 %), too. Third, this system is an ability applied to variety of opened and closed operation etc. under the keeping with condition.

## Description

### Technical Field

The present invention relates to an incubator having both a function of a clean bench used for working in an open space system and a function of the incubator used for culturing in a closed space system.

### Background Art

Generally, in culturing cells or tissues, a clean bench and incubator are used, where the clean bench provides a sterile space for an operator to manipulate cells etc. and the incubator provides a space to culture cells etc. after the operation in clean bench. The clean bench feeds sterile gas into a working space and thereby produces a sterile working space. The operator inserts hands into the working space in clean bench through an opening site to manipulate cells etc. that placed on the working space. The incubator is equipped with a sealed up the inside space, and is capable of controlling and regulating the gas composition and temperature inside incubator as desired according to experimental conditions for the culture of cells etc. placed in the incubator (Patent Literature 1). In those experiments system, the operator manipulates cells etc. using the clean bench, and then needs to culture for the cells etc. by moving them to the incubator.

However, the clean bench and incubator are separate pieces of equipment, making it necessary for the operator to take a culture vessel out of the clean bench after working on the clean bench and move the culture vessel into the incubator. Also, depending on details of the experiment, cells etc. need to be kept immersed in the culture solution having, for example, a predetermined pH level, but the pH level may change with time and cease to satisfy a predetermined culture condition. Therefore, it is necessary to constantly monitor the pH level of the culture solution and to exchange the culture solution when the measurement of pH level is departed from a predetermined value. It is so troublesome to constantly monitor and exchange the culture condition, especially, the case of that it is not desirable to exchange the culture solution by opening and closing the sealed incubator kept at a predetermined gas composition ratio because the cells etc. might not be able to be cultured appropriately.

### Citation List

### Patent Literature

Patent Literature 1: Japanese Patent Laid-Open No. 2012-90612

### Summary of Invention

### Technical Problem

The present invention has been made in view of the above problems and has an object to provide an incubator and an incubator system equipped with a clean bench function, allowing cells etc. to be manipulated and cultured.

### Solution to Problem

An incubator with a clean bench function (the first unit e.g. in this system) according to a first aspect of the present application comprises: a chamber configured to be closable and openable; a gas supply unit adapted to supply a plurality of types of gas to the chamber; a concentration control unit adapted to control concentrations of the plurality of types of gas in the chamber; and a temperature control unit adapted to control temperature in the chamber. Preferably, the chamber has an opening, and includes an inner door adapted to close the entire opening of overall, and further includes an operation cover equipped in the inner door. Preferably, the inner door further includes an operation cover fixing hole used to attach the operation cover. Preferably, the operation cover is attached to the operation cover fixing hole using a circular fixture; and the circular fixture includes two half rings pivotally connected to each other at one end and linked together by a tension spring at the other end. The operation cover has been or can be sterilized, and is an arm cover and/or glove such as a glove with intact tips, a tubular glove with the tip cut off, or an arm cover made of non-cloth paper, cloth, silicone, etc. Preferably, the chamber has an opening and includes a sealing door to close the entire opening and shut off an inside the chamber from outside air. Besides, preferably the incubator further comprises a line (in/out) port used to pull tubes into the chamber from outside the chamber. Furthermore, preferably the chamber includes: an opening, an inner door adapted to close the entire opening, a sealing door adapted to close the entire opening and shut off an inside the chamber from outside air, and an elastic material equipped between the inner door and the opening and between the sealing door and the opening; the elastic material includes a channel extending in a width direction of the elastic material; and the channel makes up a line (in/out) port used to pull tubes into the chamber from outside the chamber.

The chamber may include: an opening, an inner door adapted to close the entire opening, a sealing door adapted to close the entire opening and shut off an interior of the chamber from outside air, a first elastic material attached to the chamber, which is positioned between the inner door and the opening, a second elastic material attached to the sealing door, which is positioned between the sealing door and the opening, and a third elastic material attached to the chamber, which is positioned between the sealing door and the opening; first elastic material, second elastic material, and third elastic material may include first channels, second channels, and third channels, respectively, extending in width directions of the respective elastic materials; and first channels, second channels, and third channels may make up a line (in/out) port used to pull tubes into the chamber from outside the chamber.

Positions of first channel, second channel, and third channel may be determined such that a straight line extending along first channel, second channel, or third channel does not cross an end of another adjacent channel. The plurality of types of gas may include oxygen (O₂) gas. Also, the plurality kinds of gas may include carbon dioxide (CO₂) gas; and the gas supply unit may be equipped with a CO₂ cylinder. The plurality of types of gas may include nitrogen (N₂) gas; and the gas supply unit may be equipped with a device adapted to generate N₂ or with a N₂ cylinder. Furthermore, the plurality of types of gas may include O₂ gas and CO₂ gas; and the concentration control unit may control O₂ concentration to be 25% or less and control CO₂ concentration to be 20% or less. Besides, the plurality of types of gas may include N₂ gas; and when the incubator with a clean bench function (the first unit e.g.) is used as a clean bench, the gas supply unit may supply N₂ gas into the chamber such that positive pressure is maintained in the chamber relative to outside pressure, but when the first unit is used as an incubator, the gas supply unit does not have to supply N₂ gas into the chamber.

The temperature control unit may control in the chamber to be in the wildly range from 4 degrees Celsius in air temperature to 50 degrees Celsius adding in room temperature. The chamber may have an opening, and further include an inner door adapted to close the entire opening, an operation cover attached in the inner door, and hooks installed in the chamber and adapted to be able to catch the operation cover. The hook may be a protruding rod-shaped material inside the chamber of lateral surface curved to the back surface. The incubator may further comprise an ultraviolet (UV) lamp installed inside the chamber.

According to second invention of the present application, the incubator system with the clean bench function (the first and second units in this system) applies to be possible to operate under the condition of very low temperature (from 4 degree Celsius in air temperature) too, and applies to be possible to regulate under the different conditions of both the gas composition and temperature including 4 degree Celsius at the same time too, whereby the functions of the second unit adds to the first unit that. The second unit combined with the first unit are comprised the incubator with the clean bench function (the second unit and/or the first unit); a low temperature chamber (the second unit) adapted to keep on the inside its temperature lower than that inside the first unit; and the work chambers installed in the low temperature chamber (the second unit); and the gas control units regulated gas concentration in the work chambers (the second unit). In particular, the combined with first and second invention is possible of exchange and regulate with gas each of different temperature in this system, that is the gas control unit is possible to regulate a constituent of gas in the work chamber inside the low temperature chamber in the second unit to exchange and match a constituent of gas in the first unit. The gas control unit may control concentrations of a plurality of types of gas in the work chamber (the second unit e.g.). Also, the gas control unit may regulate a constituent of gas in the work chamber inside the low temperature chamber in the second unit to be different from a constituent of gas in the first unit. Specifically in addition, the second invention in possible of exchange and regulate with gas both of each different temperature and constituent in this system.

### Advantageous Effects of Invention

The present invention provides an incubator and an incubator system with a clean bench function (the first and second units in this system), allowing cells etc. to be manipulated and cultured.

### Brief Description of Drawings

[Figure 1] Figure 1 is a diagram schematically showing an incubator with the clean bench function (the first unit e.g.) according to a first embodiment. It is illustrate here only with the first unit, however the second unit has similar applied functions of the first unit and in addition to other functions.
[Figure 2] Figure 2 is a block diagram of the incubator with the clean bench function (the first unit e.g.).
[Figure 3] Figure 3 is a perspective view of first elastic material and third elastic material.
[Figure 4] Figure 4 is a front view of second elastic material.
[Figure 5] Figure 5 is a front view of first elastic material, second elastic material, and third elastic material with tubes passed there through.
[Figure 6] Figure 6 is a perspective view of an inner door with holes attached the membrane with slits and the operation cover to insert into the gloves for operation, and a hook, shelves, etc. inside the chamber.
[Figure 7] Figure 7 is a perspective view of a hook having another shape.
[Figure 8] Figure 8 is a diagram schematically showing an incubator system (the first and/or second units in this system) according to a second embodiment.
[Figure 9] Figure 9 is a perspective view of fourth elastic material and fifth elastic material.
[Figure 10] Figure 10 is a block diagram of the incubator system (the first and/or second units in this system).
[Figure 11] Figure 11 is a front view of a circular fixture.
[Figure 12A-D] Figure 12A is a diagram showing a technique for attaching an operation cover to the circular fixture.
[Figure 13] Figure 13 is a diagram of the operation cover as viewed from outside the inner door.

### Reference Signs List

100 Incubator with clean bench function
110 Chamber
111 Opening
114 First hook
116 UV lamp
117 Outer sealing door
118 Inner door
119 Operation cover fixing hole
120 Third elastic material
121 Second elastic material
122 First elastic material
136 Operation cover
140 Gas concentration control unit
160 Temperature control unit
170 CO₂ cylinder
180 N₂ cylinder
190 N₂ gas generator
300 Circular fixture

### Description of Embodiments

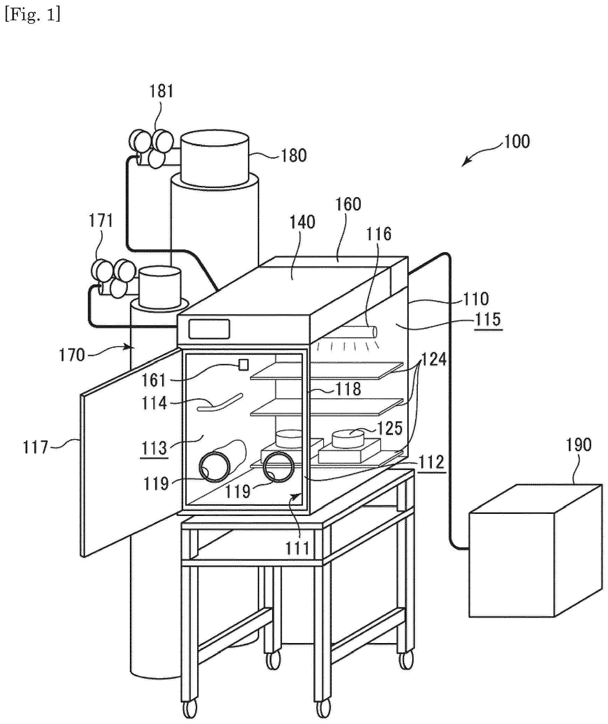

First, an incubator 100 with a clean bench function (the first unit e.g.) according to an embodiment of the present invention will be described with reference to Figures 1 to 7.

Figure 1 is a schematic diagram of the incubator 100 with a clean bench function (the first unit e.g.). The incubator 100 with a clean bench function mainly includes a chamber 110, the gas concentration control unit 140, a temperature control unit 160, a CO₂ cylinder 170, a N₂ cylinder 180, and a N₂ gas generator 190. The CO₂ cylinder 170, N₂ cylinder 180, and/or N₂ gas generator 190 makes up a gas supply unit. Note that for the sake of explanation, side faces of the chamber 110 are illustrated in such a way as to allow an inside the chamber 110 to be seen through.

The chamber 110 has the shape of a rectangular parallelepiped and includes an opening 111 formed by opening in a front of the chamber 110, an outer sealing door (sealing door) 117 and an inner door 118 attached to the opening 111, and a sensor 161. The opening 111 is a part of the front in the chamber 110, and includes a peripheral portion 112 on placement around the opening site. A first hook 114 and a sensor 161 are attached to each of two inner side surfaces 113 of the chamber 110. Note that the first hooks 114 on the other side inner surfaces 113 is omitted in Figure 1 for simplification of explanation. A detailed shape of the first hooks 114 will be described later. A UV lamp 116 adapted to emit UV rays whose wavelength is suitable for sterilization is attached on upper site of an inner back surface 115 in the chamber 110. Plural shelf boards 124 can be attached inside the chamber 110. Depth of two shelf boards 124 close to the UV lamp 116 is approximately half-long depth of the another shelf boards inside the chamber 110 and it is a reason for adjusted such that the UV rays emitted by the UV lamp 116 will easily reach any desired place inside the chamber 110. Consequently, all of the desired any place inside the chamber 110 is sterilized appropriately. The culture vessels 125 of a closed perfusion type and/or non-perfusion type etc. can be placed on the shelf boards 124. Note that a non-illustrated heat insulating material is provided on a ceiling surface, the inner back surface 115, the inner side surface 113, and a bottom surface of the chamber 110.

The sensor 161 includes a temperature sensor, CO₂ sensor, and O₂ sensor, and measures temperature, CO₂ concentration, and O₂ concentration in the chamber 110.

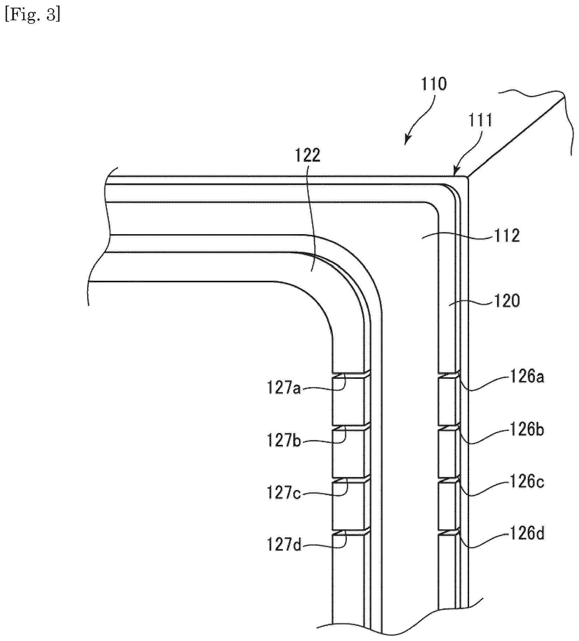

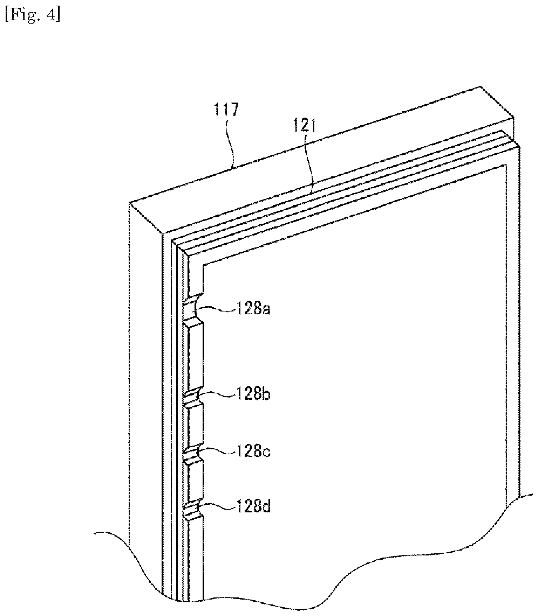

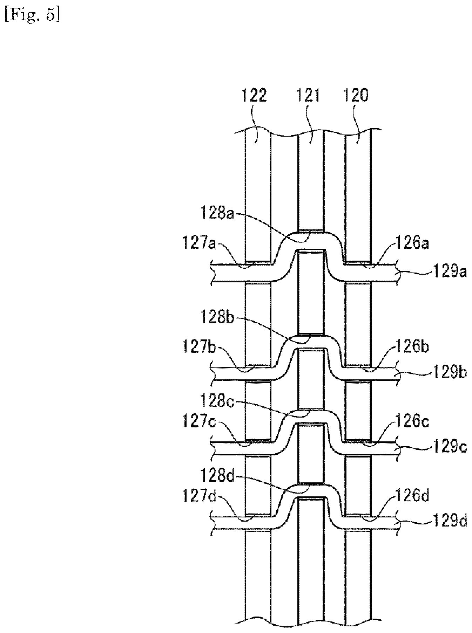

The sealing door 117 and inner door 118 are attached to the opening 111 using pivotable hinges. The inner door 118 is made of a flat transparent polycarbonate plate length enough to completely close the opening 111 and provided with two arm cover/glove fixing holes (operation cover fixing holes) 119 perforating the polycarbonate plate in a thickness direction below a vertical center. The arm cover and/or glove are referred to as an operation cover. The two operation cover fixing holes 119 each has a circular shape with the same diameter, but unnecessary of the same it. The diameter is large enough to allow human arms to pass easily, reach anywhere inside the chamber 110 easily, and work easily. First elastic material 122 (see Figure 3) is attached on the circumference inside edge in the chamber 110 at the peripheral site 112 in the chamber 110 inner door 118, where which on the most inside edge in the chamber 110 of the distance from outside edge, where is the distance from outside edge in its, and placed on the making overlapped area at the inner double doors by the closed the inner door 118 (see Figure 3, 5). Next, second elastic material 121 (see Figure 4) is attached on the circumference inside near the edge in the sealing door 117, where the located of midsection in the peripheral site 112 is made appearance of overlapped area in nearly alternated site at the few distance of outside from first elastic material 122 and inside from third elastic material 120 by closed both the sealing door 117 and the inner door118 (see Figure 5). And moreover, third elastic material 120 (see Figure 3) is attached on the outside edge of peripheral site 112 in the chamber 110, and placed on the more slightly outside second elastic material 121 above-mentioned in the overlapped area (see Figure 5). The sealing door 117 is a substantially flat-plate metal door adapted to completely close the opening 111, configured to be larger in area than the inner door 118 when viewed from the front. Details of the inner door 118, sealing door 117, third elastic material 120, second elastic material 121, and first elastic material 122 will be described later.

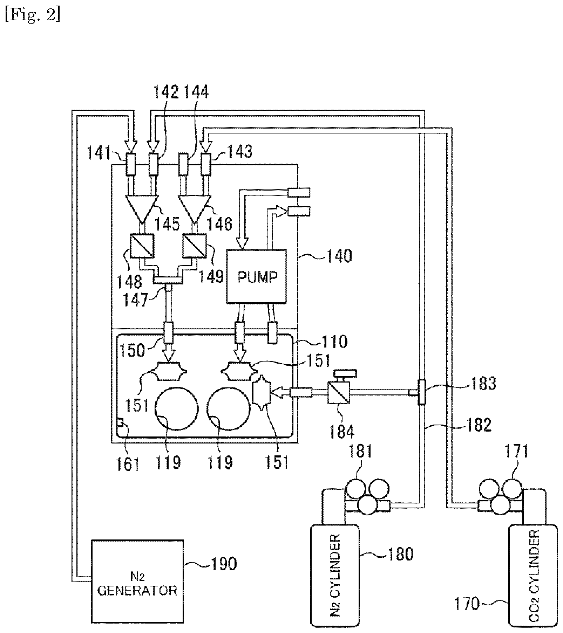

The gas concentration control unit 140 is connected with the chamber 110, CO₂ cylinder 170, N₂ cylinder 180, and N₂ gas generator 190 through tubes. The gas concentration control unit 140 adjusts CO₂ gas supplied from CO₂ cylinder 170 and N₂ gas supplied from N₂ cylinder 180 and N₂ gas generator 190 to respective predetermined concentrations, and their mixes and supplies the gas mixture into the chamber 110 (see Figure 1, 2). The concentrations of CO₂ gas and N₂ gas are adjusted such that concentrations of CO₂, N₂, and O₂ in the chamber 110 will take predetermined values. Details of the concentration control unit 140 will be described later.

The temperature control unit 160 (see Figure 1) includes a heater and a refrigerating machine and maintains predetermined temperatures inside the chamber 110, e.g., temperatures in the range from 4 degrees Celsius in air temperature to 50 degrees Celsius adding in room temperature, based on the temperature detected by the sensor 161 attached inside the chamber 110.

CO₂ cylinder 170, which is an airtight container, stores CO₂ gas. The tube is attached to CO₂ cylinder 170 via regulator 171. The regulator 171 regulates a flow rate of CO₂ gas flowing out of CO₂ cylinder 170. The tube connects CO₂ cylinder 170 to the gas concentration control unit 140. CO₂ gas is sent from CO₂ cylinder 170 to the concentration control unit 140 through the tube. N₂ cylinder 180, which is an airtight container, stores N₂ gas. The tube is attached to N₂ cylinder 180 via regulator 181. The regulator 181 regulates flow rate of N₂ gas flowing out of N₂ cylinder 180. The tube connects N₂ cylinder 180 to the gas concentration control unit 140. N₂ gas is sent from N₂ cylinder 180 to the gas concentration control unit 140 through the tube.

N₂ gas generator 190 generates N₂ gas by separating N₂ from the atmosphere. The tube connects N₂ gas generator 190 to the concentration control unit 140. N₂ gas is sent from N₂ gas generator 190 to the gas concentration control unit 140 through the tube.

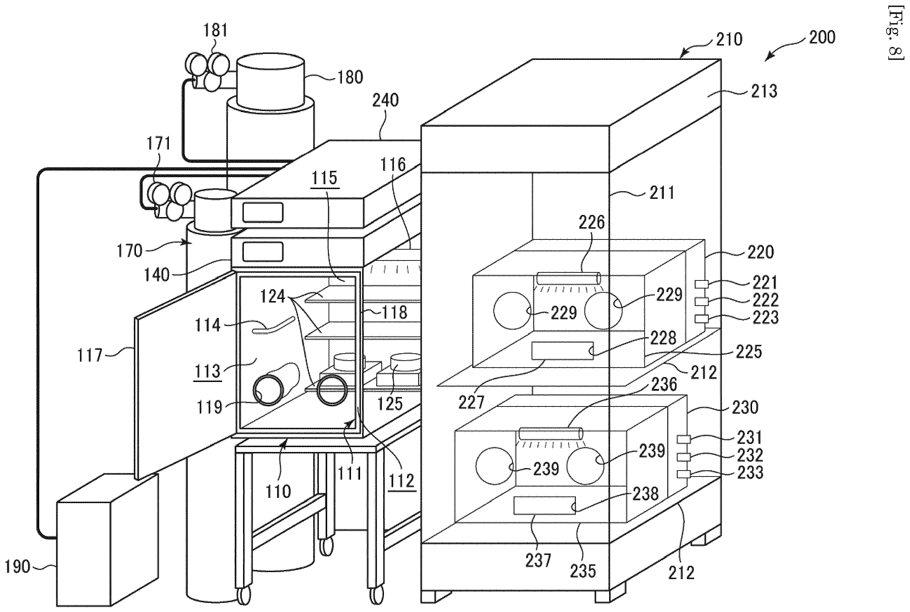

The culture solution is supplied into the chamber 110 (first and/or second units e.g.) from outside placed the chamber 220, 230, and others of chamber or non-chamber (second units e.g.) through the tubes by such a way mentioned-later (see Figure 5, 8). The culture solution stored the chamber 220, 230, and others in a non-illustrated device installed solution stored bags or containers in a work chamber 220 or 230 etc. placed in the low temperature chamber 210 (second units e.g.). The culture solution flows into the culture vessel 125 etc. inside the chamber 110 from outside placed the culture stored chamber 220, 230 etc. through tubes by the pump of syringe types or/and peristaltic types etc. which is poured into the devises while the culture solution flowing out of the culture vessel 125 etc. is discharged outside the chamber 110 through tubes. The discharged culture solution is collected by a non-illustrated device installed in a work chamber 220 or 230 and others of chamber or non-chamber etc. placed outside the chamber 110.

Next, with reference to Figure 2, detailed description will be given of the gas concentration control unit 140, and the tube connecting three-way between N₂ cylinder 180 and the control unit 140 as well as chamber 110.

The tube 182 extending from N₂ cylinder 180 is connected to the gas concentration control unit 140 and chamber 110 via a branch coupler 183. A manual valve 184 is installed between the branch coupler 183 and chamber 110. When the manual valve 184 is turned on by operator, N₂ is freely supplied in large dose from N₂ cylinder 180 into the chamber 110 through sterilization filter 151 and when the manual valve 184 is off, N₂ is not freely supplied in large dose from N₂ cylinder 180 into the chamber 110.

The concentration control unit 140 is equipped with predominantly-comprised that first to fourth connectors are 141 to 144 detachably connected with tubes, first to third mixing devices are 145 to 147 mixed with gas of two or more types gas, first and second electromagnetic valves are 148 and 149 adjusted gas pressure, and connector 150 is connected to the chamber 110. The first and second mixing devices 145 and 146 are three-way selector electromagnetic valves and third mixing device 147 is a three-way junction of tubes.

The first connector 141 is connected with tube extending from N₂ gas generator 190 while second connector 142 is connected with tube extending from N₂ cylinder 180. The first mixing unit 145 mixes N₂ gas received from N₂ gas generator 190 with N₂ gas received from N₂ cylinder 180 through first connector 141 and second connector 142, respectively, and sends the gas mixture to first electromagnetic valve 148. When the incubator 100 with a clean bench function (the first unit etc. in this system) is used as a clean bench and/or glove box, based on CO₂ and O₂ concentration measured with the sensor 161, first electromagnetic valve 148 sends N₂ gas to third mixing unit 147 at such a pressure that N₂ concentration in the chamber 110 will become equal to a predetermined concentration. On the other hand, when the first unit etc. in this system is used as an incubator, first electromagnetic valve 148 does not send N₂ gas to third mixing unit 147.

The third connector 143 is connected with tube extending from CO₂ cylinder 170. Although not connected with any tube in Figure 2, fourth connector 144 can be appropriately connected as required with CO₂ cylinder 170, CO₂ generator, or device or cylinder adapted to supply another gas. The second mixing unit 146 mixes CO₂ gas received from CO₂ cylinder 170 through third connector 143 with the gas received through fourth connector 144 and sends the gas mixture to second electromagnetic valve 149. Based on CO₂ concentration measured with the sensor 161, the second electromagnetic valve 149 sends CO₂ gas to third mixing unit 147 at such a pressure that CO₂ concentration in the chamber 110 will become equal to a predetermined concentration. CO₂ concentration in the chamber 110 is determined appropriately depending on whether first unit etc. in this system is used as the clean bench and/or glove box, or used as the incubator.

When the first unit etc. in this system is used as the clean bench and/or the glove box, the third mixing unit 147 mixes N₂ gas and CO₂ gas and sends the gas mixture into the chamber 110 through the connector 150 and sterilization filter 151. Consequently, the concentrations of N₂, CO₂, and O₂ in the chamber 110 are kept constant. On the other hand, when the first unit etc. in this system is used as the incubator, the third mixing unit 147 sends CO₂ gas into the chamber 110 through the connector 150 and sterilization filter 151, in the same way used as a clean bench and/or a glove box, above. Consequently, the concentrations of CO₂ and O₂ in the chamber 110 are kept constant. The concentration of CO₂ is possible to set from 0% to 20% and the concentration of O₂ is possible to set from 1% to 25%. That is, low O₂ partial pressure can be set as a setting condition. The concentration ranges can be selected appropriately according to the experiment.

Next, first elastic material 122 and third elastic material 120 will be described with reference to Figure 3, 5.

Third elastic material 120 is made of closed-cell foam sponge, and is attached the all circumference on the inside site of near an outer edge in the chamber 110 that part of the peripheral portion 112 which is overlapped by the closed sealing door 117, i.e.. Possible flexible materials for the closed-cell foam sponge include flexible materials such as polyurethane, chloroprene, ethylene, propylene, diene, rubber etc. Third elastic material 120 includes plurality of third channels 126a to 126d making up part of a line (in/out) port. Third channels 126a to 126d extend in a width direction of third elastic material 120, that is, in such a direction as to perforate outside and inside of the chamber 110 when the sealing door 117 is closed. Third channels 126a to 126d are installed in such a way as to be parallel to each other.

First elastic material 122 is made of a flexible material such as polyurethane, polyvinyl chloride, ethylene, propylene, rubber etc., and is attached on circumference of the peripheral portion 112 in the chamber 110, and placed on the inside than third elastic material 120. The location of first elastic material 122 is overlapped area from a slightly larger than outside of the edge in the inner door 118 to the inside edge of chamber 110 in the peripheral portion 112 by the closed inner door 118. First elastic material 122 includes plurality of first channels 127a to 127d making up part of the line (in/out) port. First channels 127a to 127d extend in a width direction of first elastic material 122, that is, in such a direction as to perforate outside and inside of the chamber 110 when the inner door 118 is closed. First channels 127a to 127d are installed in parallel to each other.

Third channels 126a to 126d and first channels 127a to 127d are installed in parallel to each other. Also, third channel 126a and first channel 127a, third channel 126b and first channel 127b, third channel 126c and first channel 127c, and third channel 126d and first channel 127d are located on the same straight lines, respectively.

Next, the second elastic material 121 will be described with reference to Figure 4, 5.

Preferably, second elastic material 121 is made of flexible material such as polyurethane, and is attached on the slightly inside circumference from outer edge in the sealing door 117, attached on such a location as to overlap the peripheral portion 112 when the sealing door 117 and the inner door 118 are closed, i.e.. Second elastic material 121 includes a plurality of second channels 128a to 128d making up part of the line (in/out) port. Second channels 128a to 128d extend in a width direction of second elastic material 121, that is, in such a direction as to perforate outside and inside of the chamber 110 when the sealing door 117 is closed. Second channels 128a to 128d are installed in parallel to each other.

Figure 5 shows the third channels 126a to 126d, second channels 128a to 128d, and first channels 127a to 127d through which tubes 129a to 129d are passed, with the inner door 118 and sealing door 117 closed. The tubes 129a to 129d are connected to a culture solution storing tanks or bags installed outside the chamber 110, and are used to make a culture solution flow in and out of a petri dish or other culture devises. Note that in the vertical direction of Figure 5, positional relationships among the third channels 126a to 126d, second channels 128a to 128d, and first channels 127a to 127d are shown in an exaggerated manner.

Positions of third channel 126a, second channel 128a, and first channel 127a are determined such that straight line extending along third channel 126a, second channel 128a, or first channel 127a will not cross an end of another adjacent channel. That is, the positions of the third channel 126a, second channel 128a, and first channel 127a are determined such that straight line extending along third channel 126a will not cross the end of the adjacent second channel 128a, that straight line extending along second channel 128a will not cross the ends of the adjacent third channel 126a and first channel 127a, and that straight line extending along first channel 127a will not cross the end of the adjacent second channel 128a. This is also true for third channels 126b to 126d, second channels 128b to 128d, and first channels 127b to 127d, and thus description thereof will be omitted. Consequently, the ends of third channels 126a to 126d, second channels 128a to 128d, and first channels 127a to 127d are not located close to each other, and thus no gas flows in and out of the chamber 110 easily through the channels. That is, inflow and leakage of gas are prevented.

The tube 129a is fitted into the third channel 126a, second channel 128a, and first channel 127a and enters the chamber 110 from outside the chamber 110. Similarly, tubes 129b to 129d enter the chamber 110 from outside the chamber 110 through third channels 126b to 126d, second channels 128b to 128d, and first channels 127b to 127d. Since the channels are provided in third elastic material 120, second elastic material 121, and first elastic material 122, the tubes 129a to 129d enter the chamber 110 without being squeezed by third elastic material 120, second elastic material 121, and first elastic material 122, allowing liquid to flow in and out of the chamber 110 easily.

In culturing cells put in petri dish or other culture devises etc., tubes already attached aseptically to the petri dish, or others before setting up the culture devises in the chamber 110 may have been made unremovable to maintain an aseptic condition. In this case, if third channels 126a to 126d, second channels 128a to 128d, and first channels 127a to 127d are used as placed with the present embodiment, the culture devises can be set up from outside to inside the chamber 110 without removing the tubes from the culture devises.

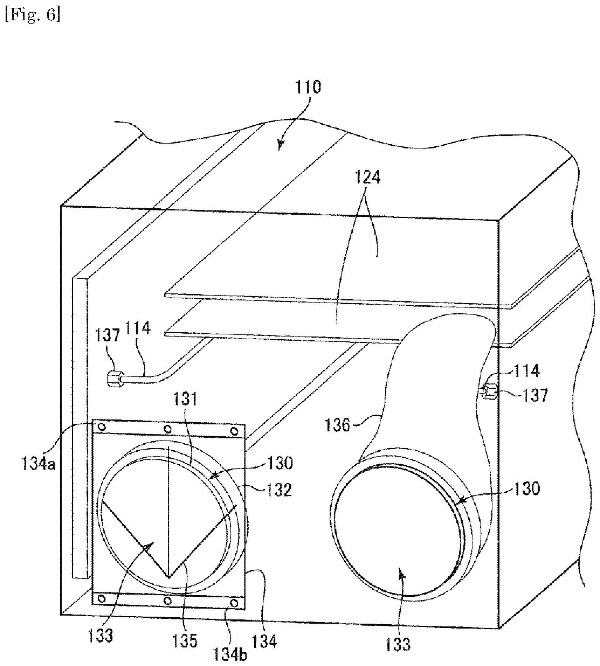

Next, the first hooks 114 and an operation cover 136 will be described with reference to Figures 6, 11, and 12A to 12D. These figures show an example in which a glove whose fingers at the tip are not cut off is used as the operation cover 136, i.e., a setup example in which an operation cover 136 of a single-layer structure is used as for the glove box, e.g..

A circular frame 130 (see Figure 6) made of silicone is attached to an inner circumferential portion of the operation cover fixing hole 119 (see Figure 1) for the operation cover 136 such as an arm cover/glove. The frame 130 includes an inner circumferential portion adapted to cover an inner circumferential surface of the operation cover fixing hole 119 and an edge portion 131 protruding slightly from a circumferential end of the inner circumferential portion in a radial direction of the operation cover fixing hole 119. A ring of disk-shaped flange 132 protruding in the radial direction of the operation cover fixing hole 119 from the inner circumferential portion at a slight distance from the inner door 118 is attached to the edge portion 131 of the frame 130. The flange 132 is coaxial with the frame 130 and is larger in maximum diameter than the frame 130.

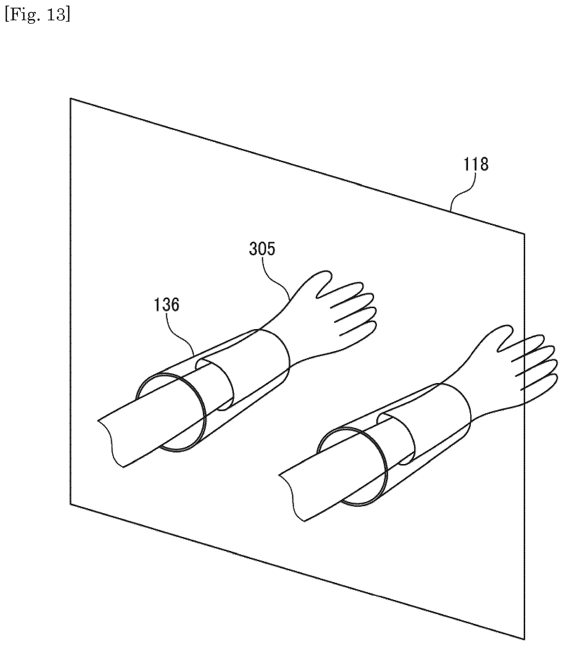

Next, when the chamber 110 is not used as a single type of glove box, in which an operation cover 136 of non-single-layer structure, such as the first unit e.g. is used as clean bench etc, the operation covers 136 is used of the double-layer structure in which a setup gloves whose fingers at the tip are cut off one and non-cut off the other, i.e., (See figure 13) A stopper 133 (see Figure 6) made of silicone rubber is fitted into the inner circumferential portion of the operation cover fixing hole 119, or to put it more specifically, into an inner circumferential portion of the circular frame 130. The stopper 133 is a disk plate shaped as a truncated cone and is inserted into the inner circumferential portion of the frame 130 with a surface with a smaller diameter foremost, bringing a circular conical surface into close contact with the inner circumferential portion of the frame 130 and thereby making it difficult for gas to flow into or out of the chamber 110 through the inner circumferential portion of the frame 130.

Also, a membrane 134 is attached on the inner door 118 to cover hole of all at an outer side of the operation cover fixing hole 119 using membrane fixture plates 134a and 134b. The membrane fixture plates 134a and 134b are plate materials made of metal. The membrane fixture plate 134a is installed above the operation cover fixing hole 119 in Figure 6 while the membrane fixture plate 134b is installed below the operation cover fixing hole 119 in Figure 6. The fixture plate 134a and 134b are fixed to the inner door 118 using plural bolts and nuts, with the membrane 134 interposed between the fixture plate 134a or 134b and inner door 118. Consequently, upper and lower sides of the membrane 134 are fixed to the inner door 118 in Figure 6, but properly, the lower side of membrane fixture plate 134b may not use. The membrane 134 is rectangular in shape, made of a silicone or a Teflon (registered trademark) material, and provided without/with slit 135 such as an arrow-shaped etc. Any side of the membrane 134 is longer than a diameter of the operation cover fixing hole 119. Consequently, the membrane 134 covers the entire operation cover fixing hole 119. The slit 135 has the shape of an arrow etc. pointed downward in Figure 6, perforating the membrane 134 in a thickness direction of the membrane 134. Note that the shape of the slit 135 can be changed appropriately according to application, and the membrane 134 may be used without any slit by being fixed only by the upper fixture plate 134a without using the lower fixture plate 134b. The operator can take a hand wearing a sterilized glove or take sterilized tools, samples etc. in and out of the chamber 110 through the slit 135, or through the lower side of the membrane 134 where the membrane 134 is not fixed membrane plate 134b when no slit is provided. In Figure 3, since the upper and lower sides of the membrane 134 are fixed to the inner door 118 with membrane fixing plate 134a and 134b, in each, upper and lower, even if the hand and tools are moved in and out through the slit 135, the membrane 134 does not cling to the hand or tools. This improves working efficiency.

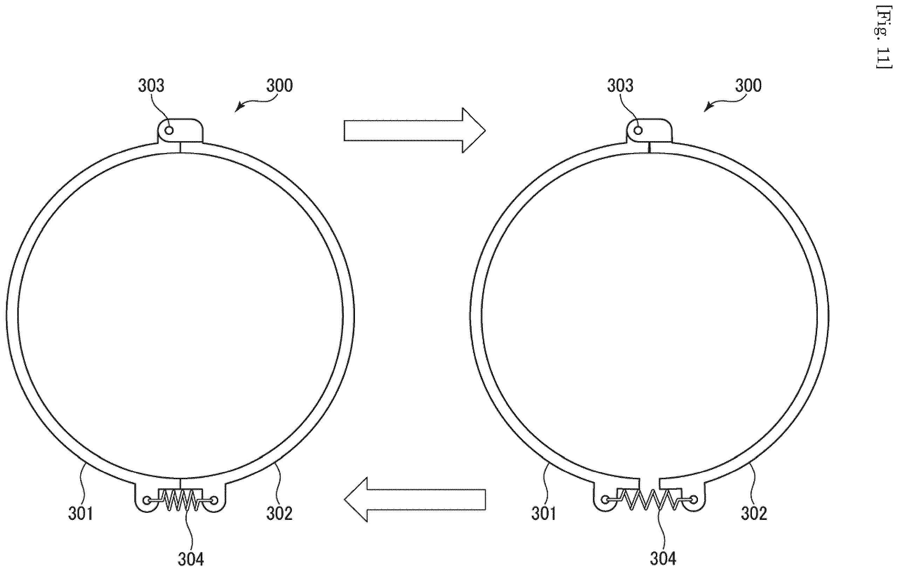

A sterilized glove with tips thereof not cut off made of a silicone material etc., a sterilized tubular glove with tips thereof cut off, or a sterilized and/or disposable tubular operation cover 136 made of non-cloth paper or etc. is attached to the flange 132. The end of the operation cover 136 is wound around the flange 132 and fixed to the flange 132 by being pinched by a circular fixture 300 attached to a fixed or removable tool of a glove and/or arm cover. Referring to Figure 11, the circular fixture 300 is made of metal which is possible to autoclave sterilization and mainly composed of half rings (301, 302), a hinge 303, and a tension spring 304. The half rings (301, 302) are jointed each other at the ends via the hinge 303 so as to be pivotable relative to each other on the hinge 303. The other ends of the half rings (301, 302) are linked to each other via the tension spring 304. The tension spring 304 applies a force in such a direction that the other ends will contact each other. As the half rings (301, 302) pivot on the jointing hinge 303, separating the ends of jointing spring 304 from each other, the ends of jointing spring 304 are brought back into contact with each other by being pulled by the tension spring 304. Consequently, the half rings (301, 302) are held pivotally in such a way as to form an annular ring. Since the half rings (301, 302) are held pivotally in such a way as to form an annular ring, the operator can easily fix and remove the circular fixture 300 within the chamber 110 with one hand.

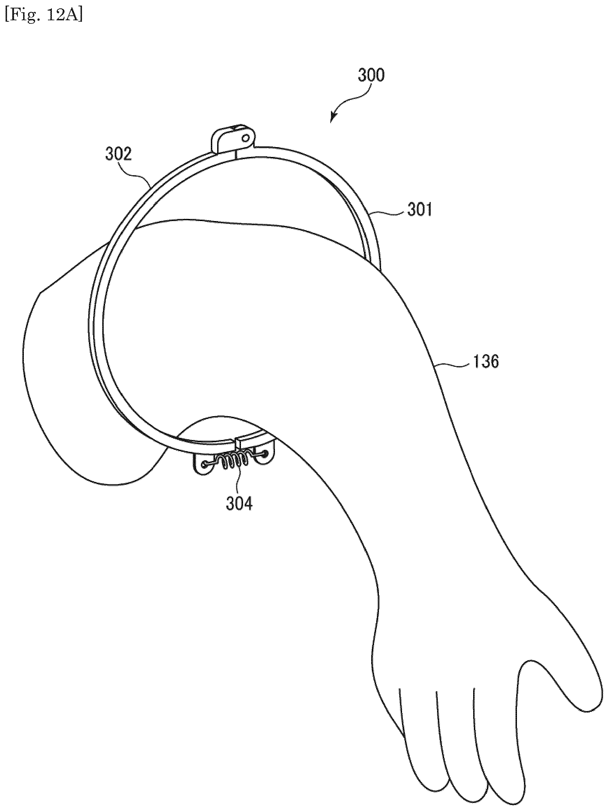

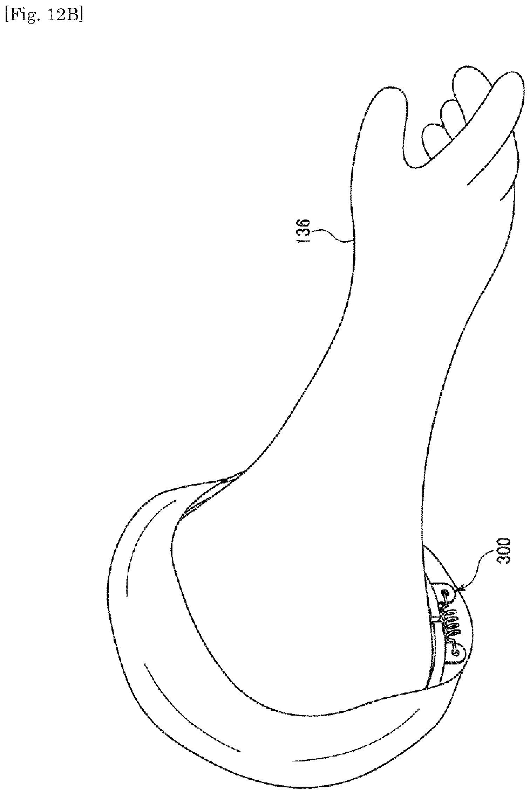

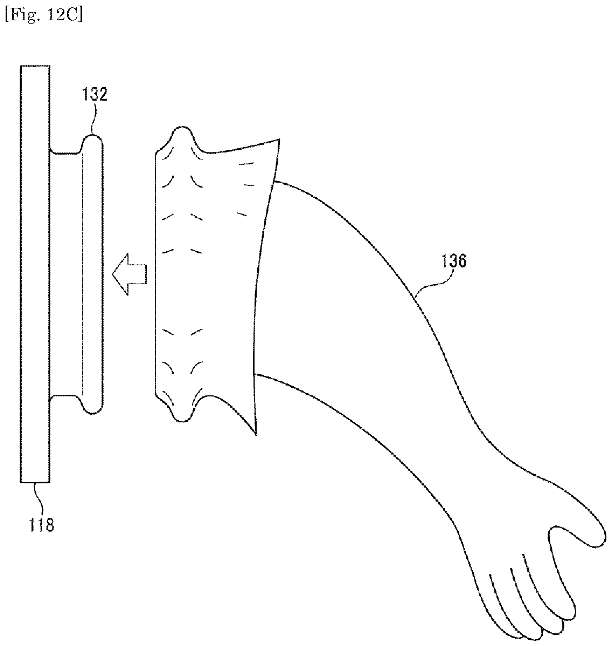

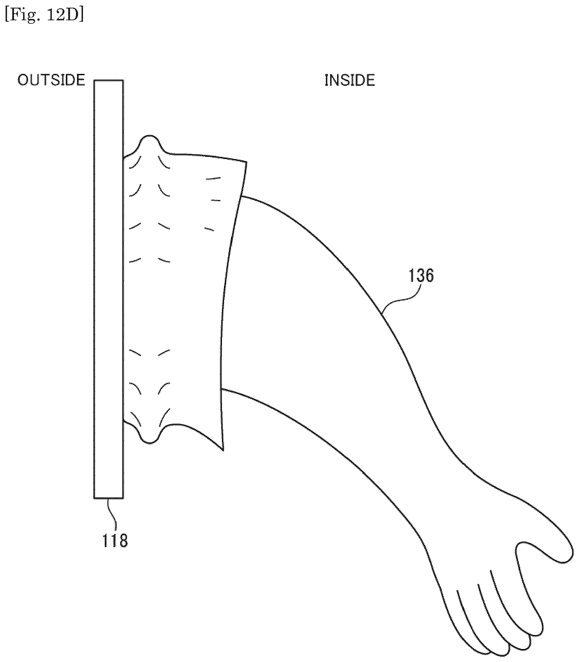

A technique for attaching the operation cover 136 to the operation cover fixing hole 119 will be described with reference to Figures 12A to 12D. Referring to Figure 12A, first the operation cover 136 is passed inside the circular fixture 300. Next, referring to Figure 12B, an opening of the operation cover 136 is turned inside out to cover the circular fixture 300 over an outer circumference of the circular fixture 300. Next, referring to Figure 12C, the operation cover 136 with the circular fixture 300 attached thereto is put on the flange 132 while opening the circular fixture 300. Next, when the operator takes off the hand from the circular fixture 300 referring to Figure 12D, the circular fixture 300 closes and gets engaged with the flange 132. Consequently, the operation cover 136 and circular fixture 300 are attached to the operation cover fixing hole 119. Note that the shape of the circular fixture 300 is not limited to the shown that in Figure 11, and the material of the circular fixture 300 does not need to be a metal. If ethylene oxide gas sterilization, ethanol sterilization (70%), or autoclave sterilization is possible, the circular fixture 300 is possible alternatives include a string-like fixture made of silicone rubber or cloth, capable of being formed into a circular shape, and equipped with a tool such as a cord stopper used to open and close a drawstring of a pouch. The fixing end in the operation cover 136 which is attached to the flange 132 is larger than the other of non-fixing end. The operation cover 136 is preferably tapered shape toward the non-fixing end with the inside chamber 100 from the fixing side of the inner door 118. When the incubator with a clean bench function (the first unit e.g.) is used as a clean bench, the operation cover 136 is attached to the flange 132 and the operator turn off the UV lamps and inserts a hand wearing a sterilized glove with the tip non-cut off into the operation cover 136, such as a tubular glove with the tip cut off or an arm cover. Since the operation cover 136 is tapered as described above, the intact glove put on the hand comes into close contact with the operation cover 136, making it difficult for gas to flow between the intact glove put on the hand and the operation cover 136. When the chamber 110 is not used as a glove box, the non-fixing end to the around of midsection in the operation cover 136 can be left hanging on the first hook 114 etc. as take care of the non-fixing end of the operation cover be left inside the chamber 100. When the around of midsection in the operation cover 136 is suspended on the hook etc., the operation cover 136 is bent back and forth by gravity. This makes it difficult for gas to flow into or out of the chamber 110 through the inside of the operation cover 136.

The first hook 114 is made of a metal rod having a circular cross section and shaped like a letter L whose corner describes an arc. The shape of the hook may be plate-like as with a second hook 138 described later, and the L-shaped corner may be square or arc-shaped, either way. The end of the first hook 114 is attached to the inner side surface 113 using a nut 137. The first hook 114 is located at such a position on the inner side surface as to allow the glove or operation cover 136 to be hung and held thereon, and moreover allow the operator to take with the inner glove worn on the hand, or the hand itself, out of the chamber 110 easily while leaving the operation cover 136 inside the clean bench by hanging from a neighborhood of the non-fixing end to the around of midsection in the operation cover 136 on the first hook 114 as described later.

A case in which an operation cover 136 of the double-layered structure is used will be described with reference to Figure 13. The operation cover 136 is made up of a sheath. The sheath is made up of glove with the tips cut off or an arm cover made of non-cloth paper, cloth, silicone material, Teflon (registered trademark) material etc., and has cylindrical shape. Preferably, the cylindrical shape is tapered, decreasing in diameter from the end of fixing site toward the end of non-fixing site. The operation cover 136 is attached to the operation cover fixing hole 119 using the above-mentioned circular fixture 300. The circular fixture 300 is attached to the end with the larger diameter. A glove 305 is inserted into an inner circumference of the operation cover 136. The glove 305 has intact tips. As the glove 305 is inserted inside the operation cover 136, a double-layered structure is realized. As described later, because large amount of N₂ gas flows into the chamber 110, maintaining positive pressure inside the chamber 110, even if the glove 305 is not put in close contact with the inner circumference of the operation cover 136, outside air does not enter the chamber 110 from outside.

Next, with reference to Figures 1, 2, and 6, description will be given of the incubator equipped with the clean bench function (the first unit e.g.), used as the clean bench and as the glove box combined with the clean bench function. Here, the tubular glove with the tip cut off (installed in the operation cover fixing hole 119 of the chamber 110) and the glove with intact tips (put on operator's hand) are used in layers as double gloves. Also, the glove box combined with the clean bench function is used as the glove box which combines the open system and the closed system. The tubular glove with the tip cut off, for which the tubular arm cover may be used, is the operation cover 136.

First, the operation cover 136 is attached to the flange 132, and the UV lamp 116 is turned on or ethanol disinfection above half-hourly is carried out to sterilize inside the chamber 110 under the condition of closed the stopper 133. Subsequently, CO₂ and N₂ gas are flowed in the chamber 110 controlled by the concentration control unit 140 until the stable of predetermined concentrations. Next step is slightly opened the stopper 133 and N₂ gas is flowed into the chamber 110 from the N₂ cylinder 180 by opening the manual valve 184. Consequently, large amount of N₂ gas flow into the chamber 110, creating positive pressure inside the chamber 110 relative to atmospheric pressure and preventing the atmosphere containing various germs from flowing into the chamber 110. While maintaining this condition, operator turn off the UV lamp 116 and open the stopper 133, and then takes pre-sterilized culture vessels and tools and container containing samples in and out of the chamber 110 through the lower side of the membrane 134 in disassembly the fixture plate 134b attached the inner door 118 or operation cover fixing hole 119 or through the slit 135 in assembly the membrane fixture plate 134a and 134b, with properly ethanol disinfection around. The samples are, for example, cells etc. in dishes made of glass and more in cooling box according to need. After the above-mentioned operations, the inner door 118 and/or operation cover fixing hole 119 is closed, the UV lamp 116 is turned on, the manual valve 184 and the stopper 133 are properly closed, and the inside of the chamber 110 going through preparatory work is sterilized. The necessity of the case is taken the samples into the chamber 110 after the above preparatory work in sterilized. Sterilized tubes used to supply and discharge the culture solution to/from the culture devise during the preparatory work are laid through the channels of the elastic materials, first 127a-127d etc and second 128a-128d etc and third 126a-126d etc, between the inner door 118 or the sealing door 117 and the chamber 110 for the passes through the inside from/to outside the chamber 110 (see Figure 5). At the time of work, operator opens the manual valve 184 and the stopper 133, turns off the UV lamp 116, inserts the hand wearing the sterilized glove with intact tips into the operation cover 136, and manipulates the samples, the culture medium etc. When the manipulations are finished, operator closes the gloved hand into a fist such that glove fingers which touched the culture medium, samples etc. will be placed inside the palm of the glove worn on the hand. And then, operator hangs from a neighborhood of the non-fixing end to the around of midsection in the operation cover 136 on the hook 114 by moving the arm and elbow, with the back of the up, in such a way that fingers will not touch the hook 114. The next step, operator pulls the hand together with the glove out of the operation cover 136 while taking care inside its(136) circumference of no-touch with the glove fingers grasping hands which touched the culture medium, samples etc. Then, after the exchange of the glove worn on the hand with a new sterilized glove outside the chamber 110, operator closes the operation cover fixing hole 119 with the stopper 133 properly sterilized with ethanol (70%), turns on the UV lamp 116, and closes the manual valve 184.

At the time of clean bench work, as above-mentioned, just before starting the work, with a large amount of N₂ gas allowed to flow into the chamber 110 by opening the manual valve 184, operator takes pre-sterilization of the culture vessel and the tools and container containing samples in and out mainly through the lower side of the membrane 134 with free from the fixture plate 134b attached the operation cover fixing hole 119 or through the slit 135 while minimizing the duration, opening degree, and frequency of opening the inner door 118. Consequently, changes in the concentrations and temperatures of O₂ and CO₂ in the chamber 110 being used as a clean bench are minimized.

For the non-impairment of function in the clean bench, it is honked or alarm of warning sound in the cases of deviation from the range of advance preparation setting on each O₂ or CO₂ concentration in this system. In the cases of honked of warning sound or the ringing on ahead, the stopper 133 is opened/closed or the change of opening degree in the stopper 133 with rotation angles among the operation caver fixing hole 119 is controlled by the breaking through gas between the stopper 133 and the operation cover fixing hole 119, while opening the manual valve 184 and with properly repeating above sterilization.

For the safety and protect for operator against infection etc., the time on working inside the chamber 110 or after-mentioned chamber 220, 230 in this system is essential that UV lamp 116 turn off and operator wears a mask etc. and sterilized disposable and non-permeability of gloves for the non-contact with samples or culture buffer and so on.

Regarding how to hold the vessel/container when taking in and out sterilized the culture vessel or the tools and container containing samples, similarly the vessel/container is taken in and out by being held with the gloved hand closed into a fist with the fingers of the glove worn on the hand turned inward or the vessel/container is set up inside the chamber 110 through the operation cover 136 via the lower side of the membrane 134 or via the slit 135 by holding the vessel/container or tool from above, with the back of the hand describing an arc such as when a container such as a tea caddy for powdered green tea is held during tea ceremony. Also, in taking a tool or vessel/container out of the chamber 110 together with the glove worn on the hand, the neighborhood of the end of the operation cover 136 is hung on the hook 114 or the like by moving the arm and elbow without using fingertips and the work is carried out in a similar manner.

Also, in pulling the glove worn on the hand out of the chamber 110 at the end of a culture operation etc., if the glove fingers which touched the culture medium, samples, or are fear in probability to have touched, an inner side of an operation cover 136, in the case of long-term culture, the operator carries out disinfection by turning on the UV lamp 116 not less than a half-time with closed the manual valve 184 and the stopper 133. And then, operator wears a new sterilized glove and mask outside the chamber 110, leaves in the membrane 134 and opens the manual valve 184 and the stopper 133 again, and turns off the UV lamp 116, inserts the hand wearing the new sterilized glove into the chamber through the operation cover fixing hole 119 under the lower side of the membrane 134 for the non-polluted operation cover 136 (on one side) which did not touch the fingers of glove which had touched the culture medium, samples etc. In next, operator takes off the polluted operation cover 136 (on the other side) with the unopened inside polluted site in which touched the fingers of glove that had touched the culture medium, samples etc., from the circular fixture frame 130 include the flange 132, and speedily encloses it in using sterilization bags, and exchange the new operation cover 136 with a sterilized and prepared beforehand inside the chamber 110. After the change of this, operator turn on the UV lamp 116 and closed the stopper 133 and the manual valve 184. Note that if a contaminated glove touches the membrane 134 or the slit 135 in the membrane 134, similarly the membrane 134 can be easily replaced with a sterilized new membrane 134 by taking off the fixture plate 134a. The present embodiment not only makes it easy to exchange gloves while carrying out work or continuing culture, which is difficult with the conventional glove box, but also makes it possible to exchange of all commodities, such as the membrane 134 and operation cover 136 etc., on any route through which tools and samples containers etc. pass from the inside of the chamber 110 to the outside of the inner door.

What has been described above is only an example, and depending on culture conditions, the tubes used to supply and discharge culture solutions may become unnecessary; and under conditions in which O₂ and CO₂ concentrations are close to atmospheric pressure or during work in which things are taken in and out of the box for a shorter duration or less frequently, the operation cover 136 is unnecessary, and the membrane 134 and slit 135 provided in the operation cover fixing hole 119 and inflow of large amount of N₂ gas into the box 110 caused by opening the manual valve 184 may be sufficient.

Next, with reference to Figures 1, 2, and 6, description will be given of the incubator 100 equipped with the clean bench function (the first unit e.g.) used as the glove box. Here, description will be given of the embodiment which uses the single-glove with intact tips and uses this system of function as the closed-system glove box which does not require gloves and tools to be exchanged or replenished during culture. Specifically, it is the case of non-using the function of clean bench in culturing stage, exclude of preparation stage etc., and so the manual valve 184 and the inner door 118 and the stopper 133 etc. are all closed in culturing stage.

First, the glove with intact tips is attached as an operation cover 136 to the operation cover fixing hole 119 via the flange 132, UV lamp 116 is turned on, and ethanol disinfection is carried out to sterilize inside the chamber 110. The manual valve 184 is opened, allowing large amount of N₂ gas to flow into the chamber 110 from N₂ cylinder 180, and then the stopper 133 is slightly opened. Next, UV lamp 116 is turned off, and then culture vessels and tools and samples are carried and placed in the chamber 110 for preparation of culture. In the culture stage, the inner door 118 or the stopper 133 of the operation cover fixing hole 119 is closed, the UV lamp 116 is turned on if objects to be cultured is not affected, and the manual valve 184 is closed. In the case of apprehensive affected objects by UV lamp 116, it is necessary is placed on the samples under the foil-wrapped cover or container, or properly turn off the UV lamp 116. When the culture vessels and tools and samples are set up in preparation, tubes used to supply and discharge the culture solution to/from the samples are laid through the channels of the elastic materials, first 127a-127d etc and second 128a,-128d etc and third 126a-126d etc, between the inner door 118 or the sealing door 117 and the chamber 110 for the passes through the inside from/to outside the chamber 110 (see Figure 5). If it is not necessary to perfuse the culture medium, the pipes used to supply and discharge a culture solution may not be laid.

When the inner door 118 is opened or closed before the start of culture or after the end of culture, if the duration, opening degree, and frequency of opening/closing the inner door 118 are minimized with large amount of N₂ gas being allowed to flow into the chamber 110 by opening the manual valve 184, the temperatures and concentrations of O₂ and CO₂ are maintained as described above. While maintaining this condition, the operator carries out work with the UV lamp 116 turned off during the work. In the treatment of warning samples and culture medium and materials etc. that suspected to affect human bodies by substances of harmful bacteria or toxic reagents etc. in which desired to be avoided flowing out of the chamber 110 in vapor phase and others, note that if the samples or the reagents is taken out of an enclosed airtight container in the chamber 110, the manual valve 184 is kept closed, and the pump 154 etc. which for vapor phase exchange between the first work chamber 220 described later and the chamber 110 is switched off and shut down. Operator works inside the chamber 110 from gloves 136 with intact tips on the operation cover fixing holes 119 , and after the work, operator put out the hands to keep the gloves inside the chamber 110 and UV lamp 116 is turned on to disinfect the inside the chamber 110. In the work beginning in preparation, there are doing the operation of setting up the container with the sample and reagent enclosed therein in the chamber 110, and laying the tubes used to supply and discharge the culture solution in/from the inner door 118, and ending with the operation of sterilizing the exterior of the container inside the box with UV lamp 116, which is similar to the work described above.

Consequently, the exchange of the culture solution can be supplied by perfusion in/out the culture solution while maintaining the culture condition including, temperatures, O₂ and CO₂ concentration etc. and the culture solution can be supplied from outside system 100 without opening/closing the inner door 118 or the operation cover fixing hole 119 in the present system 100. For the safety and protect of operator against infection etc., and for the care of honked or alarm of warning sound on gas control, it is described as the preceding paragraph <0047>.

Furthermore, if there is concern about safety of the operator, the sample can be manipulated using the double-glove structure made of gloves with intact tips by inserting a hand wearing the sterilized glove with intact tips into the glove 136 with intact tips, the glove 136 being fixed to the chamber 110. It is possible to use this system for globe box in non-using the function of clean bench not only culturing stage but also preparation and cleanup after the culture.

Next, with reference to Figures 1, 2, and 6, description will be used as the incubator function instead of non-using clean bench function in this system of the incubator 100 equipped with a clean bench (the first unit e.g.). In this case, samples manipulated using the clean bench function and glove box function is already placed in the chamber 110 and the inner door 118 is closed. In this condition, the inner circumferential stopper 133 has been fitted in the operation cover fixing hole 119 and the sealing door 117 has been closed. At this time, tubes used to supply and discharge a culture solution to/from the samples have been laid among third channels 126a to 126d, second channels 128a to 128d, and first channels 127a to 127d (see Figure 5). The temperatures and O₂ concentration and CO₂ concentration are maintained constant as described above by making N₂ gas and CO₂ gas flow into the chamber 110 as required. While maintaining this condition, the culture solution is supplied to the sample through the tubes to culture the samples. Regarding the condition during culture, the inside of the culture vessel is observed from outside the transparent inner door 118 using a microscope with a variable observation angle with the culture vessel remaining set up inside the chamber 110. Since microscopic observation can be conducted without opening the inner door 118 or the stopper 133 of the operation cover fixing hole 119, even for consecutive observation during culture, there is no need to take the culture vessel out of the incubator chamber 100, the temperatures and concentrations of O₂ and CO₂ are maintained constant, and there is less stress on the culture samples. Alternatively, it is also possible to observe the condition of culture in the culture vessel inside the chamber 110 by inserting a microscope objective lens shaped like a fiber cable etc. used for endoscopy etc. through the channels 126 in sealing materials between inner door 118, and through the lower side of the membrane 134 on free from the membrane fixing plate 134b attached on the outside of the operation cover fixing hole 119 or through the slit 135 via the operation cover 136 attached to the flange 132 by simultaneously combination using the clean bench function of allowing large amount of N₂ gas to flow into the chamber 110 from N₂ cylinder 180 by opening the manual valve 184 and slightly opening the stopper 133 in adjusted time.

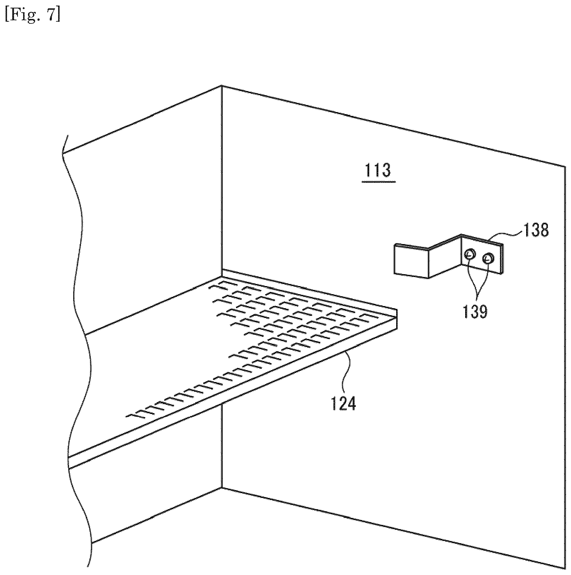

Next, the second hook 138 having another shape will be described with reference to Figure 7. The second hook 138 is made of plate-like metal and shaped like a letter S whose corners are square. The hook has the same uses as the first hook 114, and thus may have a shape similar to that of the first hook 114. The end of one side in the second hook 138 is attached to the inner side surface 113 using the bolt 139. The position of the second hook 138 on the inner side surface 113 is similar to that of the first hook 114, and thus description thereof will be omitted.

According to the present embodiment, after being manipulated, cells etc. can be cultured without moving the culture vessel 125 out of a clean bench, i.e., out of the chamber 110. Also, the culture environment which satisfies the low-temperature or low O₂ condition can be created easily.

Next, an incubator system 200 (the second unit e.g.) according to the second embodiment will be described with reference to Figures 8 to 10. Components similar to those of the first embodiment in the first unit e.g. are denoted by the same reference numerals as the corresponding components, and description thereof will be omitted. The present embodiment in the second unit e.g. differs from the first embodiment in that the incubator system 200 further includes a low temperature chamber 210, the first work chamber 220, the second work chamber 230, and gas control unit 240. Mainly these components will be described below.

Figure 8 is a schematic diagram of the incubator system 200 (the second units e.g.). The incubator system 200 mainly includes the incubator 100 with a clean bench function (the first and/or second unit in this system), the low temperature chamber 210 (the second unit e.g.), the first work chamber 220 (the second unit e.g.), the second work chamber 230 (the second unit e.g.), and the gas control unit 240 (the second unit e.g.). Note that for the sake of explanation, the chamber 110 (the first and/or second unit e.g,), low temperature chamber 210, first work chamber 220, and second work chamber 230 are illustrated in such a way as to allow inside these components to be seen through.

The low temperature chamber 210 has the shape of rectangular parallelepiped and includes left-hand (which in possible of right-hand, too) front door 211 attached to the front of the low temperature chamber 210 and made of transparent polycarbonate plate, heater, and refrigerating machine 213. The heater and refrigerating machine 213 maintains predetermined temperatures inside the low temperature chamber 210, e.g., temperatures in the range from 4 degree Celsius in air temperature to 50 degrees Celsius adding in room temperature, based on the temperature detected by a non-illustrated temperature sensor attached inside the low temperature chamber 210. The refrigerating machine is used, for example, for a range from 4 degrees Celsius in air temperature (inclusive) to a neighborhood of room temperature, and the heater is used, for example, for a range from a neighborhood of room temperature to 50 degrees Celsius adding in room temperature (inclusive). The room temperature is, for example, from 25 to 27 degrees Celsius. One or more shelf boards 212 are installed inside the low temperature chamber 210. The first and second work chambers 220 and 230 are possible to set up on the shelf board(s) 212.

The first work chamber 220 has the shape of a rectangular parallelepiped and mainly includes the opening 261 formed by opening in the front of the first work chamber 220, the right-hand (which in possible of right-hand, too) front door 225 attached to the opening 261 using pivotable hinges, the UV lamp 226, and the sensor 269. The front door 225 is made of flat transparent polycarbonate plate about large enough to completely close the opening 261 and provided with the rectangular hole 228 perforating the polycarbonate plate in the thickness direction below the vertical center and two arm cover/glove fixing holes (the operation cover fixing hole) 229 perforating the polycarbonate plate in the thickness direction above the vertical center. The rectangular hole 228 has a width and height about large enough to pass the culture vessel 125. The upward-opening rectangular door 227 is attached to the rectangular hole 228. The rectangular door 227 is provided with non-illustrated locking mechanism to prevent the rectangular door 227 from opening inadvertently. The sensor 269 includes temperature sensor, CO₂ sensor, and O₂ sensor, and measures temperature, CO₂ concentration, and O₂ concentration in the first work chamber 220.

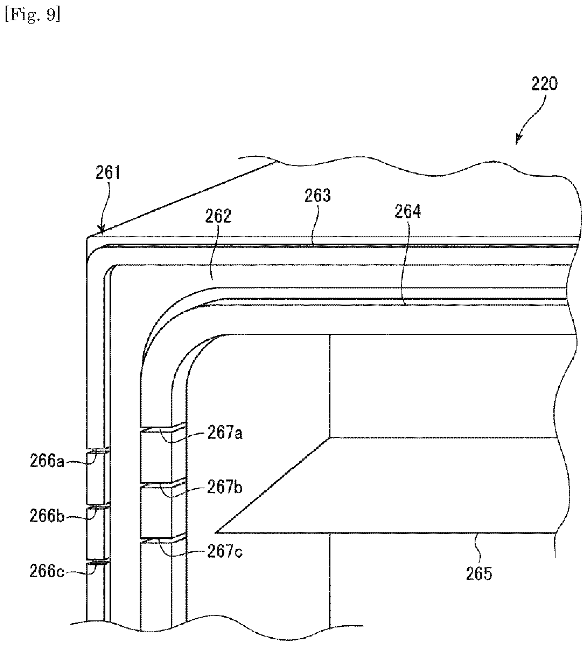

The configuration of the two operation cover fixing holes 229 is similar to that of the operation cover fixing holes 119 according to the first embodiment, and thus description thereof will be omitted. The UV lamp 226 is attached to the inner back surface of upper site in the first work chamber 220 and emits UV rays whose wavelength is suitable for sterilization. The hook similar to the first hook 114 or second hook 138 is attached and plural shelf boards 265 (see Figure 9) can be attached inside the first work chamber 220. Configurations of the first hook 114, second hook 138, UV lamp 226, and shelf board 265 are similar to those of the first embodiment, and thus description thereof will be omitted. Also, the first hook 114 or second hook 138 is omitted in Figure 9 for simplicity of explanation. The non-illustrated heat insulating material is attached the ceiling surface, an inner back surface, an inner side surface, and the bottom surface of the first work chamber 220. Fourth elastic material 263 and fifth elastic material 264 (see Figure 9) are attached that part of peripheral portion 262 which is overlapped by the closed front door 211.

The first work chamber 220 mainly includes seventh to ninth connectors 221 to 223 detachably connected with tubes, fifth electromagnetic valve 224 adapted to adjust gas pressure, and seventh mixing unit 268 adapted to mix plural types of gas. Seventh mixing unit 268 is three-way junction tube. Operations of these components will be described later.

Next, fourth elastic material 263 and fifth elastic material 264 will be described with reference to Figure 9.

Fourth elastic material 263 is preferably made of closed-cell foam sponge of flexible material such as consist of polyurethane, chloroprene, ethylene, propylene, diene, rubber etc. and is attached on the entire outer edge in the chamber 220 that part of the peripheral portion 262 which is overlapped by the closed front door 225. Fourth elastic material 263 includes plurality of fourth channels 266a to 266d making up part of the line (in/out) port. Fourth channels 266a to 266d extend in width direction of fourth elastic material 263, that is, in such a direction as to perforate outside and inside of the first work chamber 220 when the front door 225 is closed. Fourth channels 266a to 266d are installed in such way as to be parallel to each other.

Fifth elastic material 264 is preferably made of the flexible material such as consist of polyurethane, vinyl chloride, ethylene, propylene, rubber etc. and is attached on the entire inner edge in the chamber 220 that part of the peripheral portion 262 which is overlapped by the closed front door 225. Fifth elastic material 264 includes a plurality of fifth channels 267a to 267d making up part of the line (in/out) port. Fifth channels 267a to 267d extend in width direction of fifth elastic material 264, that is, in such a direction as to perforate outside and inside of the first work chamber 220 when the front door 225 is closed. Fifth channels 267a to 267d are installed in parallel to each other. The fourth channels 266a to 266d and fifth channels 267a to 267d are installed in parallel to each other. Also, fourth channel 266a and fifth channel 267a, fourth channel 266b and fifth channel 267b, fourth channel 266c and fifth channel 267c, and fourth channel 266d and fifth channel 267d are located on different straight lines, respectively. Consequently, ends of fourth channels 266a to 266d and fifth channels 267a to 267d are not located close to each other, and thus no gas flows in and out of the first work chamber 220 easily through the channels. That is, inflow and leakage of gas are prevented.

The second work chamber 230 mainly includes an upward-opening front door 235 and a UV lamp 236, where the front door 235 includes a rectangular hole 238, two operation cover fixing holes 239, and a sensor 279. The configuration of the second work chamber 230 is similar to that of the first work chamber 220, and thus description thereof will be omitted. The second work chamber 230 mainly includes 20th to 22nd connectors 231 to 233 detachably connected with tubes, sixth electromagnetic valve 234 adapted to adjust gas pressure, and an eighth mixing unit 278 adapted to mix plural types of gas. Eighth mixing unit 278 is a three-way junction tubes. Operations of these components will be described later. The sensor 279 includes temperature sensor, CO₂ sensor, and O₂ sensor, and measures temperature, CO₂ and O₂ concentration in the second work chamber 230.

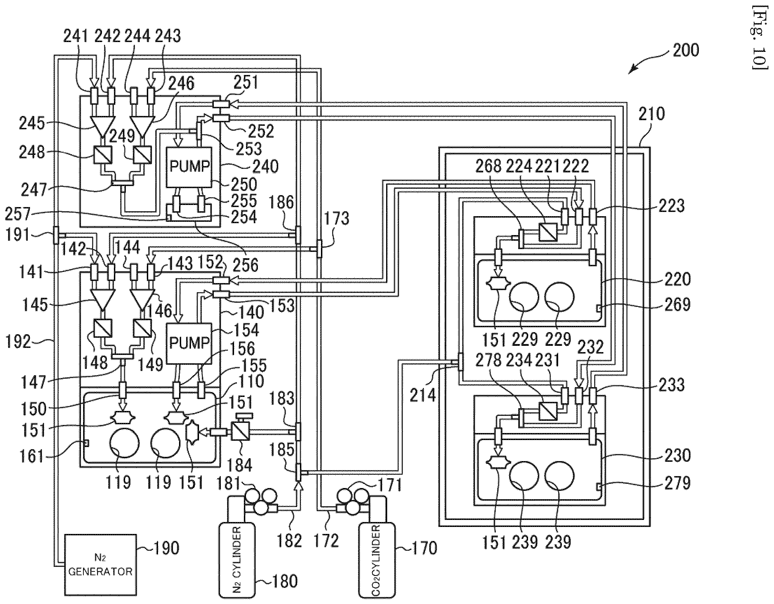

Next, the tubes used to connect various materials as well as the gas control unit 240 will be described in detail with reference to Figure 10.

The tube 182 extending from N₂ cylinder 180 is connected to the gas control unit 240 via branch couplers 185, 183, and 186; connected to the first work chamber 220 and second work chamber 230 via branch couplers 185 and 214; connected to the chamber 110 via branch couplers 185 and 183; and connected to the concentration control unit 140 via the branch couplers 185, 183, and 186.

The tube 192 extending from N₂ gas generator 190 is connected to the concentration control unit 140 and gas control unit 240 via a branch coupler 191.

The tube 172 extending from CO₂ cylinder 170 is connected to the concentration control unit 140 and gas control unit 240 via a branch coupler 173.

The concentration control unit 140 mainly includes fifth and sixth connectors 152 and 153 detachably connected with tubes, the first pump 154, and seventh and eighth connectors 155 and 156 adapted to interconnect the first pump 154 and chamber 110.

Fifth and sixth connectors 152 and 153 are connected with tubes extending from the first work chamber 220. First pump 154 aspirates gas out of the first work chamber 220 via ninth connector 223 and fifth connector 152 and sends out the sucked gas into the chamber 110 via the eighth connector 156. On the other hand, the first pump 154 sucks gas out of the chamber 110 via seventh connector 155 and sends out the sucked gas into the first work chamber 220 via sixth connector 153. In the first work chamber 220, the 18th connector 222 receives gas from the first pump 154 and sends the gas to seventh mixing unit 268. Seventh mixing unit 268 sends the gas into the first work chamber 220 through the sterilization filter 151. Consequently, the first work chamber 220 contains the same gas composition as in the chamber 110. When the first work chamber 220 is used as clean bench or glove box, N₂ gas is further supplied to the first work chamber 220 from N₂ cylinder 180 via the branch couplers 185 and 214. In the first work chamber 220, the 17th connector 221 receives N₂ gas from N₂ cylinder 180 and sends N₂ gas to the fifth electromagnetic valve 224. Based on CO₂ concentration and O₂ concentration measured with the sensor 269 installed inside the first work chamber 220, fifth electromagnetic valve 224 sends N₂ gas to the seventh mixing unit 268 at such a pressure that N₂ concentration in the first work chamber 220 will become equal to the predetermined concentration. Seventh mixing unit 268 sends out N₂ gas into the first work chamber 220 through the sterilization filter 151. This creates the positive pressure inside the first work chamber 220 relative to atmospheric pressure and thereby prevents the atmosphere containing various germs from flowing into the first work chamber 220.

The gas control unit 240 mainly includes ninth to twelfth connectors 241 to 244 detachably connected with tubes, fourth to sixth mixing units 245 to 247 adapted to mix plural types of gas, third and fourth electromagnetic valves 248 and 249 adapted to adjust gas pressure, second pump 250, the subtank 256 adapted to accumulate gas, thirteenth and fourteenth connectors 251 and 252 adapted to connect the second pump 250 to the second work chamber 230, 15th and 16th connectors 254 and 255 adapted to connect the second pump 250 to the subtank 256. The fourth and fifth mixing units 245 and 246 are three-way selector electromagnetic valves and the sixth mixing unit 247 is the three-way junction tube. A sensor 257 is attached in the subtank 256. The sensor 257 includes CO₂ sensor and O₂ sensor and measures CO₂ and O₂ concentration in the subtank 256.

Ninth connector 241 is connected with tube extending from N₂ gas generator 190 and tenth connector 242 is connected with tube extending from N₂ cylinder 180. Fourth mixing unit 245 mixes N₂ gas received from N₂ gas generator 190 with N₂ gas received from N₂ cylinder 180 through ninth connector 241 and tenth connector 242, respectively, and sends the gas mixture to the third electromagnetic valve 248. When the second work chamber 230 is used as the clean bench or a glove box, based on the CO₂ concentration and O₂ concentration measured with the sensor 279 installed inside the second work chamber 230, third electromagnetic valve 248 sends N₂ gas to the sixth mixing unit 247 at such a pressure that N₂ concentration in the second work chamber 230 will become equal to the predetermined concentration. The N₂ cylinder 180 supplies N₂ gas to the second work chamber 230 via the branch coupler 185, branch coupler 214, and twentieth connector 231. In the second work chamber 230, the twentieth connector 231 receives N₂ gas and sends N₂ gas to sixth electromagnetic valve 234. Based on CO₂ and O₂ concentration measured with the sensor 279 installed inside the second work chamber 230, the sixth electromagnetic valve 234 sends N₂ gas to the eighth mixing unit 278 at such a pressure that N₂ concentration in the second work chamber 230 will become equal to the predetermined concentration. Eighth mixing unit 278 sends out N₂ gas into the second work chamber 230 through the sterilization filter 151. This creates positive pressure inside the second work chamber 230 relative to atmospheric pressure and thereby prevents the atmosphere containing various germs from flowing into the second work chamber 230. On the other hand, when the second work chamber 230 is used as an incubator, third electromagnetic valve 248 does not send N₂ gas to sixth mixing unit 247.

The 11th connector 243 is connected with tube extending from CO₂ cylinder 170. Although not connected with any tubes in Figure 10, twelfth connector 244 can be appropriately connected as required with CO₂ cylinder 170, CO₂ generator, or device or cylinder adapted to supply another gas. The fifth mixing unit 246 mixes CO₂ gas received from CO₂ cylinder 170 through the 11th connector 243 with the gas received through the twelfth connector 244 and sends the gas mixture to the fourth electromagnetic valve 249. Based on CO₂ concentration measured with the sensor 279 installed inside the second work chamber 230, the fourth electromagnetic valve 249 sends CO₂ gas to the sixth mixing unit 247 at such a pressure that CO₂ concentration in the second work chamber 230 will become equal to the predetermined concentration. The CO₂ concentration in the second work chamber 230 is determined appropriately depending on whether the incubator 100 with a clean bench function is used as the clean bench or the glove box, or used as the incubator.

When the second work chamber 230 is used as the clean bench or the glove box, the sixth mixing unit 247 mixes N₂ gas and CO₂ gas and sends the gas mixture to branch coupler 253.

The second pump 250 sucks gas out of the second work chamber 230 via the 22nd connector 233 and 13th connector 251 and sends out the sucked gas into the subtank 256 via the 15th connector 254. On the other hand, the second pump 250 sucks gas out of the subtank 256 via the 16th connector 255 and sends out the sucked gas to the branch coupler 253. At this time, the gases mixed by the sixth mixing unit 247 have been sent out to the branch coupler 253. Consequently, the gas mixed by the sixth mixing unit 247 and the gas sucked out of the gas subtank 256 are mixed by the branch coupler 253 and sent to the second work chamber 230 via the 14th connector 252 and 21st connector 232. The second work chamber 230 sends the gas received by the 21st connector 232 to the eighth mixing unit 278. The eighth mixing unit 278 mixes N₂ gas received from the sixth electromagnetic valve 234 with the gas received from the 21st connector 232 and sends the gas mixture into the second work chamber 230 through the sterilization filter 151.

Consequently, when the second work chamber 230 is used as the clean bench or the glove box, the concentrations of N₂, CO₂, and O₂ in the second work chamber 230 are kept constant and positive pressure is created inside the second work chamber 230 relative to atmospheric pressure, thereby preventing the atmosphere containing various germs from flowing into the second work chamber 230.

On the other hand, when the second work chamber 230 is used as the incubator, the sixth mixing unit 247 sends CO₂ gas into the second work chamber 230 via the branch coupler 253. Consequently, the concentrations of CO₂ and O₂ in the second work chamber 230 are kept constant. The concentration of CO₂ is capable of setting up 0% to 20% and the concentration of O₂ is capable of setting up 1% to 25%. The concentration ranges can be selected appropriately according to the experiment.

The present embodiment provides the first work chamber 220 containing the gas of the same composition as in the chamber 110. Also, the present embodiment provides the second work chamber 230 containing the gas of composition different from that in the chamber 110.

By saving the culture medium in the second work chamber 230 and manipulating and culturing cells in the first work chamber 220, it is possible to save the culture medium and manipulate and culture the cells while keeping the culture medium sterile.

Note that the position to which the UV lamp 116 is attached is not limited to the inner back surface 115. The position suitable for sterilizing the interiors of the chamber 110, first work chamber 220, and second work chamber 230 can be selected appropriately.

Note that the temperature inside the chamber 110 is not limited to the range from 4 degrees Celsius in air temperature to 50 degrees Celsius adding in room temperature, and may be selected appropriately according to the experiment.

Note that only N₂ cylinder 180 may be used without using N₂ gas generator 190. Also, although description has been given of the configuration in which N₂ gas and CO₂ gas are supplied into the chamber 110 and controlled, only one type of gas or more and another additional gas, such as O₂ gas etc., each experiment desired additional type of gas may be supplied into the chamber 110 and controlled. In that case, the connector, the mixing unit, and the electromagnetic valve are installed appropriately.

Note that the third channels 126a to 126d, second channels 128a to 128d, and first channels 127a to 127d do not need to have substantial widths such as shown in Figures 3 to 5 and may be shaped as narrow so-called slits. Also, the extending directions of the third channels 126a to 126d, second channels 128a to 128d, and first channels 127a to 127d are not limited to the directions described above, as long as the directions are parallel to the front of the inner door 118 or sealing door 117 and cross a pivot axis of the inner door 118 or sealing door 117. Also, third channels 126a to 126d do not have to be parallel to each other, and similarly second channels 128a to 128d do not have to be parallel to one another, and first channels 127a to 127d do not have to be parallel to each other. Furthermore, third channels 126a to 126d, second channels 128a to 128d, and first channels 127a to 127d do not have to be parallel to each other.

Third channel 126a and first channel 127a, third channel 126b and first channel 127b, third channel 126c and first channel 127c, and third channel 126d and first channel 127d may be located on different straight lines, respectively.

Also, regarding the positional relationships among third channels 126a to 126d, second channels 128a to 128d, and first channels 127a to 127d in the vertical direction of Figure 5, the channels may be placed at closer positions than shown in Figure 5. Then, the tubes 129a to 129d will be bent more gently, allowing the culture solution to flow easily in the tubes 129a to 129d. Furthermore, in Figure 5, the tubes 129a to 129d may be installed in third channels 126a to 126d, second channels 128a to 128d, and first channels 127a to 127d in a staircase pattern. That is, for example, the tube 129d is installed in third channel 126d, second channel 128d, and first channel 127c. Consequently, the tubes 129a to 129d are bent gently, allowing the culture solution to flow easily in the tubes 129a to 129d.

Note that fourth channels 266a to 266d and fifth channels 267a to 267d do not need to have substantial width such as shown in Figure 9, and may be shaped as narrow so-called slits. Also, the extending directions of fourth channels 266a to 266d and fifth channels 267a to 267d are not limited to the directions described above, as long as the directions are parallel to the front of the front doors 225 and 235 and cross pivot axes of the front doors 225 and 235. Also, fourth channels 266a to 266d do not have to be parallel to each other and similarly fifth channels 267a to 267d do not have to be parallel to each other. Furthermore, fourth channels 266a to 266d and fifth channels 267a to 267d do not have to be parallel to each other.

Fourth channel 266a and fifth channel 267a, fourth channel 266b and fifth channel 267b, fourth channel 266c and fifth channel 267c, and fourth channel 266d and fifth channel 267 may be located on the same straight lines, respectively.

The positions of first channels 127a to 127d, second channels 128a to 128d, third channels 126a to 126d, fourth channels 266a to 266d, and fifth channels 267a to 267d are not limited to the positions described above, and may be provided on entire peripheries of first elastic material 122, second elastic material 121, third elastic material 120, fourth elastic material 263, and fifth elastic material 264.

Note that the positions of the operation cover fixing holes 119 are not limited to positions below the vertical center of the inner door 118 and the positions of the operation cover fixing holes 229 and 239 are not limited to positions above the vertical center of the inner door 118.

The culture vessel 125 may be test tube, tapper, bottle, petri dish, flask, or another container of any of various shapes. Although the closed-system culture method using the perfusate has mainly been described in the present embodiment, the present device is also applicable to the culture method which does not use the perfusate, the culture method which uses the open system, and the culture method which uses combination of the open system and the closed system. Also, in these culture method, the shape of culture vessel is not limited to the one described above.

The sensor 161 may further include N₂ sensor to measure N₂ concentration in the chamber 110. When the incubator 100 with the clean bench function is used as the clean bench or the glove box, based on N₂ concentration measured with the sensor 161, the first electromagnetic valve 148 may send N₂ gas to the third mixing unit 147 at such a pressure that N₂ concentration in the chamber 110 will become equal to the predetermined concentration.

Note that the sensor 269 may further include N₂ sensor to measure N₂ concentration in the first work chamber 220. Based on N₂ concentration measured with the sensor 269 installed inside the first work chamber 220, fifth electromagnetic valve 224 may send N₂ gas to seventh mixing unit 268 at such a pressure that N₂ concentration in the first work chamber 220 will become equal to a predetermined concentration.

Note that the sensor 279 may further include N₂ sensor to measure N₂ concentration in the second work chamber 230. Based on N₂ concentration measured with the sensor 279 installed inside the second work chamber 230, the third electromagnetic valve 248 may send N₂ gas to sixth mixing unit 247 at such a pressure that N₂ concentration in the second work chamber 230 will become equal to a predetermined concentration. Also, based on N₂ concentration measured with the sensor 279 installed inside the second work chamber 230, sixth electromagnetic valve 234 may send N₂ gas to eighth mixing unit 278 at such a pressure that N₂ concentration in the second work chamber 230 will become equal to the predetermined concentration.

Note that the materials of the components described above are not limited to those described above.

The operation cover 136, which has been or can be sterilized, is preferably the glove with intact tips, the tubular glove with the tip cut off, or the arm cover made of non-cloth paper, cloth, silicone, etc. The tubular glove with the tip cut off and the arm cover are preferably tapered in the chamber, but are possible to be otherwise tapered. The operation cover made of non-cloth paper, cloth, silicone, etc. may be shaped such that one tip and/or both tips of the tube, or midsection of the tube will be squeezed by rubber etc.

Note that if a strictly sterilized condition is not required, the operator may insert a bare hand disinfected with ethanol etc. into the chamber using only the sheath as the operation cover 136 without using the glove 305 on the inner side. Furthermore, if the incubator function is unnecessary or if conditions close to atmospheric pressure are enough, the case of only clean bench function of allowing flow with large amount of N₂ gas into the chamber 110 and thereby creating positive pressure inside the chamber 110 may be used without using the operation cover 136 either.

Note that the numbers which represent the sizes of various materials shown in the present specification and the drawings are exemplary and not restrictive.

Whereas embodiments of the present invention have been described with reference to the accompanying drawings, it will be apparent to those skilled in the art that changes can be made to structures and relationships of various parts without departing from the spirit and scope of the invention set forth in the appended claims.

## Claims

1. An incubator accompanying a clean bench function comprising:
a chamber configured to be closable and openable;
a gas supply unit adapted to supply plurality of types of gas to the chamber;
a concentration control unit adapted to control concentrations of the plurality of types of gas in the chamber; and
a temperature control unit adapted to control temperature in the chamber.

2. The incubator accompanying the clean bench function according to claim 1, wherein the chamber has an opening, and further includes an inner door adapted to close the entire opening and an operation cover attached on the inner door.

3. The incubator accompanying the clean bench function according to claim 2, wherein the inner door further includes an operation cover fixing hole used to attach the operation cover.

4. The incubator accompanying the clean bench function according to any one of claims 1 to 3, wherein the chamber has the opening, and includes a sealing door adapted to close the entire opening and shut off the inside the chamber from outside air.

5. The incubator accompanying the clean bench function according to any one of claims 1 to 4, further comprising a line (in/out) port used to pull a tube into the chamber from outside the chamber.

6. The incubator accompanying the clean bench function according to any one of claims 1 to 5, wherein:
the chamber includes:
the opening,
the inner door adapted to close the entire opening,
the sealing door adapted to close the entire opening and shut off the inside chamber from outside air,
an elastic material attached between the inner door and the opening and between the sealing door and the opening;
elastic material includes a channel extending in the width direction of the elastic material; and
the channel makes up the line (in/out) port used to pull the tube into the chamber from outside the chamber.

7. The incubator accompanying the clean bench function according to any one of claims 1 to 6, wherein:
the chamber includes:
the opening,
the inner door adapted to close the entire opening,
the sealing door adapted to close the entire opening and shut off inside the chamber from outside air, and
first elastic material attached to the chamber, which is positioned between the inner door and the opening in the chamber,
second elastic material attached to the sealing door, which is positioned between the sealing door and the opening in the chamber, and
third elastic material attached to the chamber, which is positioned between the sealing door and the opening in the chamber;
first elastic material, second elastic material, and third elastic material include first channel, second channel, and third channel, respectively, extending in width directions of the respective elastic materials; and
first channel, second channel, and third channel make up the line (in/out) port used to pull the tube into the chamber from outside the chamber.

8. The incubator accompanying the clean bench function according to claim 7, wherein positions of first channel, second channel, and third channel are determined such that a straight line extending along first channel, second channel, or third channel does not cross an end of another adjacent channel.

9. The incubator accompanying the clean bench function according to any one of claims 1 to 8, wherein the plurality of types of gas include O₂ gas.

10. The incubator accompanying the clean bench function according to any one of claims 1 to 9, wherein: the plurality of types of gas include CO₂ gas; and the gas supply unit is equipped with a CO₂ cylinder.

11. The incubator accompanying the clean bench function according to any one of claims 1 to 10, wherein: plurality of types of gas include N₂ gas; and the gas supply unit is equipped with a device adapted to generate N₂ gas or with a N₂ cylinder.

12. The incubator accompanying the clean bench function according to any one of claims 1 to 11, wherein: plurality of types of gas include O₂ gas and CO₂ gas; and the concentration control unit controls O₂ concentration to be 25% or less (1 to 25 % e.g.) and controls CO₂ concentration to be 20% or less (0 to 20 % e.g.).

13. The incubator accompanying clean bench function according to any one of claims 1 to 12, wherein: plurality of types of gas include N₂ gas; and when the incubator accompanying clean bench function is used as the clean bench, the gas supply unit supplies in large quantity N₂ gas into the chamber such that positive pressure is maintained in the chamber relative to outside pressure, but when the incubator accompanying clean bench function is used as the incubator, the gas supply unit does not supply in large quantity N₂ gas into the chamber.

14. The incubator accompanying the clean bench function according to any one of claims 1 to 13, wherein the temperature control unit controls in the chamber to be in wildly range from 4 degrees Celsius in air temperature to 50 degrees Celsius adding in room temperature.

15. The incubator accompanying the clean bench function according to any one of claims 1 to 14, wherein the chamber has the opening, and further includes the inner door adapted to close the entire opening, the operation cover attached on the inner door, and a hook installed in the chamber and adapted to be able to hang the operation cover.

16. The incubator accompanying the clean bench function according to claim 15, wherein the hook is a rod-shaped material protruding from the inner side surface of the chamber and bending toward the inner back surface.

17. The incubator accompanying the clean bench function according to any one of claims 1 to 16, further comprising a UV lamp installed inside the chamber.

18. The incubator system comprising:
the incubator accompanying the clean bench function according to claim 1;
a low temperature chamber adapted to keep on the inside temperature lower than that the temperature inside the incubator accompanying the clean bench function according to claim 1;
a work chamber installed in the low temperature chamber; and
the gas control unit adapted to control gas concentration in the work chamber.

19. The incubator system according to claim 18 wherein the gas control unit controls the constituent of gas inside the work chamber to match a constituent of gas inside the incubator accompanying the clean bench function.

20. The incubator system according to claim 19 wherein the gas control unit exchanges the gas inside the incubator accompanying the clean bench function with the gas inside the work chamber.

21. The incubator system according to claim 18 wherein the gas control unit regulates the concentration in plurality types of gas in the work chamber.

22. The incubator system according to claim 21 wherein the gas control unit regulate the constituent of gas in the work chamber to be different from a constituent of gas in the incubator with the clean bench function.

23. The incubator with the clean bench function according to claim 3, wherein the operation cover is attached to the operation cover fixing hole using a circular fixture; and the circular fixture includes half rings pivotally connected to each other at one end and linked together by a tension spring at the other end.
